# DEMANDE DE BREVET EUROPEEN

(11) **EP 1 013 659 A2**
(43) Date de publication de la demande: **28.06.2000**
(21) Numéro de dépôt: 99403023.7
(22) Date de dépôt: 03.12.1999
(51) Int. Cl.: C07H 3/02

(54) **Procédé de préparation d'un aldose ou dérivé d'aldose par décarboxylation**

(30) Priorité: 04.12.1998 FR 9815342
(71) Demandeur: ROQUETTE FRERES, 62136 Lestrem (FR)
(72) Inventeur: Tamion, Rodolphe, 62157 Allouagne (FR)
(74) Mandataire: Boulinguiez, Didier

(57) **Abrégé**

La présente invention a pour objet un procédé de fabrication d'un aldose ou d'un dérivé d'aldose comportant sur la chaîne hydrocarbonée n atomes de carbone, caractérisé par le fait que l'on met en contact, en phase aqueuse, au moins un dérivé acide d'ose à n + 1 atomes de carbone comportant au moins un motif α-hydroxyacide, et/ou au moins un sel d'un tel dérivé acide d'ose, avec du peroxyde d'hydrogène en présence d'une quantité d'au moins un sel de tungstène ou de molybdène, ladite quantité étant inférieure à 4 équivalents, de préférence inférieure à 2 équivalents, exprimée en nombre total de mole(s) de tungstène et molybdène divisé par le nombre total de mole(s) de dérivé(s) acide(s) d'ose et de sel(s) de dérivé(s) acide(s) d'ose.

## Description

La présente invention a pour objet un procédé de fabrication d'un aldose ou d'un dérivé d'aldose.

Plus précisément, elle a pour objet un procédé de fabrication d'un aldose ou d'un dérivé d'aldose comportant sur la chaîne hydrocarbonée n atomes de carbone, à partir d'un dérivé acide d'ose à n+1 atomes de carbone présentant au moins un motif α-hydroxyacide, et/ou d'un de ses sels, ce procédé consistant à mettre en contact ledit dérivé acide d'ose avec du peroxyde d'hydrogène (eau oxygénée) en présence d'au moins un sel de métal choisi dans le groupe constitué par le tungstène et le molybdène, en phase aqueuse.

Au sens de la présente invention, on convient d'entendre par :
- « aldose », un ose comportant une fonction aldéhyde, en particulier un tétrose ou un pentose comportant une telle fonction, de préférence choisi parmi l'érythrose, le thréose, le ribose, le xylose et l'arabinose ;
- « dérivé d'aldose », plus particulièrement un acide uronique (acide monocarboxylique dérivé d'un aldose par remplacement du groupement CH₂OH par un groupement COOH) ;
- « dérivé acide d'ose comportant au moins un motif α-hydroxyacide », un acide mono- ou dicarboxylique dérivé d'un aldose, comportant une fonction CHOH en a de la ou des fonction(s) acide(s) et se présentant sous forme libre et/ou lactonisée.

Le procédé de la présente invention permet d'obtenir, avec une excellente sélectivité, un aldose à partir d'un acide aldonique, ou un acide uronique à partir d'un acide aldarique, sous forme libre, lactonisée et/ou de sel(s).

Les aldoses obtenus par la mise en oeuvre du procédé conforme à l'invention sont d'un grand intérêt en tant que tels, mais seraient surtout des intermédiaires de synthèse très importants s'il advenait que l'on puisse les produire en grande quantité et à faible coût. En effet, une étape complémentaire d'hydrogénation de ces aldoses permet d'obtenir facilement les alditols correspondants, qui sont tous des polyols pouvant être employés dans de multiples applications et notamment en tant que substituts non cariogènes et hypocaloriques du saccharose.

Les acides uroniques obtenus par la mise en oeuvre du procédé conforme à l'invention, en tant qu'acides polyhydroxycarboxyliques, possèdent des propriétés séquestrantes pouvant être exploitées dans le domaine des ciments, mortiers ou bétons où de tels acides ont été proposés comme retardateurs de prise, ou encore dans le domaine de la détergence, où ces acides ont été proposés pour le nettoyage d'articles en verre ou en métal ou à titre d'additifs pour détergents.

C'est en étudiant la méthode exposée par RUFF, il y a près d'un siècle (Ber. 32, 3674 (1889) ; 33, 1799 (1900)) que la Demanderesse a mis au point ce nouveau procédé de fabrication d'un aldose ou d'un dérivé d'aldose, par voie chimique, à partir d'un dérivé acide d'ose et/ou de ses sels.

La méthode de RUFF permet de passer, de façon générale, d'un acide aldonique comportant n carbones à un aldose comportant (n-1) carbones grâce à l'action combinée d'ions ferriques et d'eau oxygénée. Cependant, les rendements en aldose sont très médiocres.

La conversion de l'acide gluconique en D-arabinose peut ainsi être réalisée selon cette méthode.

Quelques améliorations ont, par la suite, été apportées par R.C. Hockett et C.S. Hudson (J. Amer. Chem. Soc. 56, 1632-1633, (1934) et ibid. 72, 4546, (1950)) et par les inventeurs du brevet US-A 3.755.294. Des rendements de 60 % d'arabinose en partant d'acide gluconique y sont décrits. Un progrès a encore été accompli par V. Bilik (CZ - 232647, (1983)) en utilisant les ions cuivriques (Cu (II)) comme catalyseurs. Des rendements de l'ordre de 70 % sont atteints après une purification laborieuse. Sont notamment nécessaires l'addition de résines échangeuses d'ions et une chromatographie sur colonne.

Des résultats identiques ont été obtenus récemment avec un mélange d'ions ferriques et ferreux comme catalyseurs (CZ - 279002, (1994)).

Enfin, dans des conditions particulières, le document EP-A 0.716.067 rapporte des rendements de 78 % en certains aldoses, mais la technique nécessite également une chromatographie échangeuses d'ions afin d'éliminer les impuretés.

Durant une investigation approfondie de la réaction de Ruff, la Demanderesse a découvert que si la réaction est catalysée par des concentrations sélectionnées de sels de tungstène ou de molybdène (tous deux métaux du groupe VI), on peut, de façon inattendue, résoudre les problèmes de sélectivité qui accompagnaient les procédés antérieurs et empêchaient leur développement.

Des complexes du tungstène sont bien cités comme catalyseurs d'oxydation des alcools secondaires en cétones en présence d'eau oxygénée (S.E. Jacobson et al., J ; Org. Chem, 44, 921 - 924, (1979)) ou de l'amidon et des maltodextrines en oligomères possédant des fonctions acides (M. Floor, Starch / Staërke, 41, 303 - 309, (1989)) ou encore comme agent d'époxydation d'alcènes ou d'oxydation des alcools primaires et secondaires en aldéhydes et cétones (K. Sato, Bull. Chem. Soc. Jpn, 70, 905 - 915, (1997)).

Les performances comparatives de nombreux sels, dont le tungstate de sodium, comme catalyseurs de l'oxydation d'hydrates de carbone, dont la glucono-delta-lactone (« GDL »), par l'eau oxygénée a été largement décrite par M. R. EVERETT et F. SHEPPARD dans « Oxidation of Carbohydrates, Salt Catalysis », University of Oklahoma Medical School, 1944, pp. 25-90. Cependant, les quantités de sels envisagées sont très élevées, à savoir généralement de plusieurs « équivalents d'ions », i.e. de plusieurs moles de l'ion étudié (anion ou cation) par mole d'hydrate de carbone.

Compte tenu de sa faible efficacité, notamment en regard de l'anion bicarbonate qui est présenté comme deux fois plus efficace conformément au second paragraphe de la page 44 dudit document, l'anion tungstate est mis en oeuvre (sous forme de tungstate de sodium) à raison de 4 équivalents en vue de catalyser l'oxydation, par l'eau oxygénée, de GDL apportée sous forme extrêmement diluée (solution à 1%). Les résultats présentés au niveau des tableaux VII page 42 et IX page 51 dudit document ne permettent cependant pas de connaître la nature et la concentration exactes du ou des produit(s) résultant de l'oxydation ainsi menée en présence de tungstène. En tout état de cause, les enseignements généraux de ce document vont à l'encontre de l'utilisation de moins de 4 équivalents d'ions tungstène, soit, compte tenu de la masse moléculaire de ce métal (environ 184), de moins de 4 x 184, soit 736 g environ de tungstène, par mole d'hydrate de carbone à oxyder. Par ailleurs, ce document n'envisage pas spécifiquement l'usage de sels de molybdène en vue de catalyser l'oxydation d'hydrates de carbone par l'eau oxygénée.

Par ailleurs le brevet FR 2 346 451 envisage, en toute généralité et sans l'exemplifier, la possibilité d'utiliser, entre autres catalyseurs, des oxydes de molybdène dans le cadre de l'oxydation de l'acide gluconique en arabinose. Cependant, il n'apparaît pas sans ambiguïté du passage situé page 3, lignes 17-26 dudit brevet avec quel(s) oxydant(s) les auteurs pensent possible d'associer lesdits sels de molybdène, et notamment s'il s'agit de l'eau oxygénée (peroxyde d'hydrogène), dont l'usage est exemplifié au niveau de l'exemple 2 et/ou d'un acide comme l'acide peracétique (cf. exemple 8) ou l'acide perbenzoïque (cf. exemple 9). En tout état de cause, les auteurs préconisent tout particulièrement l'usage combiné de l'eau oxygénée et d'ions Fe³⁺ et donc d'une méthode de RUFF classique.

Donc, aucun des documents précités ne décrit, ni ne suggère réellement l'intérêt de l'utilisation d'un système particulier sel de tungstène / eau oxygénée ou sel de molybdène / eau oxygénée pour la réaction de décarboxylation oxydative d'un dérivé acide d'ose, ou un de ses sels, en phase aqueuse. Rien, en particulier, ne suggère qu'il soit possible d'augmenter considérablement la sélectivité de ladite réaction, ce qui se traduit par la diminution remarquable de la production des coproduits tels que l'acide formique.

Cette nouvelle catalyse possède donc un potentiel important et il n'existe pas, à la connaissance de la société Demanderesse, de réaction équivalente.

De manière surprenante et inattendue, la société Demanderesse a également constaté qu'il est possible de réduire de manière importante la durée et d'augmenter encore significativement la sélectivité de la réaction catalysée par les sels de tungstène ou de molybdène en leur associant des agents dopants, tels certaines espèces métalliques, en particulier certains sels métalliques, à base de métaux autres que le tungstène et le molybdène, notamment à base de cuivre. A ce propos, la société Demanderesse a trouvé que des sels de cuivre comme les sulfates, acétates, ou chlorures de cuivre, à une concentration comprise entre 0,0001 et 1% de préférence comprise entre 0,001 et 0,5% convenaient parfaitement. A titre d'exemple, on choisit 0,05% de sel(s) de cuivre. Ces valeurs s'entendent par rapport au poids sec de dérivé(s) acide(s) d'ose et de sel(s) de dérivé(s) acide(s) d'ose mis en oeuvre. De tels agents dopants peuvent être amenés par de l'eau non déminéralisée utilisée en lieu et place de tout ou partie de la composante aqueuse du milieu réactionnel.

Un autre avantage du procédé conforme à l'invention est que comparativement aux procédés de l'art antérieur, il est possible de mettre moins d'eau oxygénée en oeuvre dans le milieu réactionnel.

Ainsi, selon l'invention, le procédé de fabrication d'un aldose ou d'un dérivé d'aldose comportant sur la chaîne hydrocarbonée n atomes de carbone, est caractérisé par le fait que l'on met en contact, en phase aqueuse, au moins un dérivé acide d'ose à n+1 atomes de carbone comportant au moins un motif α-hydroxyacide, et/ou au moins un sel d'un tel dérivé acide d'ose avec du peroxyde d'hydrogène en présence d'une quantité d'au moins un sel de tungstène ou de molybdène, inférieure à 4 équivalents, de préférence inférieure à 2 équivalents, exprimée en nombre total de mole(s) de tungstène et molybdène divisé par le nombre total de mole(s) de dérivé(s) acide(s) d'ose et de sel(s) de dérivé(s) acide(s) d'ose.

Le procédé conforme à l'invention met donc en oeuvre un dérivé acide d'ose et/ou un de ses sels. Dans la présente invention, comme indiqué précédemment, on entend par dérivé acide d'ose, un acide mono- ou dicarboxylique dérivé d'un aldose. Cette définition englobe en particulier :
- les acides aldoniques, qui sont des acides monocarboxyliques dérivés des aldoses par remplacement du groupement aldéhyde par un groupement carboxy, tels que les acides gluconique, glucoheptonique, mannonique, idonique, gulonique, galactonique, lyxonique, xylonique, arabinonique, ribonique, maltobionique et lactobionique, et en particulier l'acide gluconique, sous forme libre et/ou lactonisée, la forme lactonisée pouvant avantageusement consister en glucono-δ-lactone (GDL),
- les acides aldariques, qui sont des acides dicarboxyliques dérivés des aldoses par remplacement des deux groupements terminaux (CHO et CH₂OH) par des groupements carboxy, tels que les acides glucarique, galactarique, arabinarique et xylarique,
- les acides uroniques qui sont des acides monocarboxyliques dérivés des aldoses par remplacement du groupement terminal CH₂OH par un groupement carboxy, étant entendu que ces produits ne sont pas les dérivés acides d'ose préférés dans le cadre de l'invention.

Dans la présente invention, on entend par « sel d'un dérivé acide d'ose », un produit choisi dans le groupe comprenant les sels et esters de tout dérivé acide d'ose tel que défini ci-avant et les mélanges quelconques d'au moins deux de ces produits.

Ainsi, par exemple, les sels alcalins et alcalino-terreux, en particulier les sels de calcium et de sodium, de ces dérivés acides d'oses conviennent parfaitement. Il peut s'agir notamment des sels de calcium ou de sodium de l'acide gluconique.

La Société Demanderesse a trouvé que l'on pouvait avantageusement mettre en oeuvre au moins un dérivé acide d'ose, sous forme libre et/ou lactonisée, au sein d'un mélange contenant également au moins un sel, de préférence un sel alcalin ou alcalino-terreux, d'un dérivé acide d'ose.

En tant que substrats pouvant être mis en contact avec du peroxyde d'hydrogène conformément à l'invention, de tels mélanges se sont révélés aptes à améliorer encore les sélectivité et rendement d'oxydation en regard de substrats constitués uniquement de dérivés acides d'ose, sous forme libre et/ou lactonisée.

Il peut s'agir tout particulièrement, comme exemplifié ci-après, de mélanges contenant de la GDL et au moins un sel de l'acide gluconique, de préférence du gluconate de sodium ou de calcium.

Le poids sec de sel(s) de dérivé d'acide d'ose au sein de tels mélanges peut notamment représenter de 0,5 à 20%, de préférence de 1 à 10%, du poids sec de dérivé(s) acide(s) d'ose contenu dans de tels mélanges.

A titre d'exemple, il peut s'agir d'un mélange à base de GDL et de 2,5% environ de gluconate de sodium, ce pourcentage étant exprimé en poids sec de gluconate de sodium sur le poids sec de GDL.

Selon une autre variante de l'invention, le sel de dérivé acide d'ose, par exemple le sel d'acide aldonique, peut être substitué, en tout ou partie, par un sel, notamment alcalin ou alcalino-terreux, d'un acide autre que d'un dérivé acide d'ose tel qu'un sel de l'acide acétique, par exemple de l'acétate de sodium ou un sel d'un acide sulfonique aromatique ou aliphatique, par exemple du paratoluène sulfonate de sodium.

Les acides aldoniques sont obtenus de manière connue par oxydation de l'ose correspondant. Cette étape d'oxydation peut être conduite soit par voie chimique, soit par voie microbiologique.

La voie chimique préférée dans le cadre de l'invention consiste à oxyder l'ose à l'aide d'air ou d'oxygène en milieu alcalin et à l'aide de catalyseurs au palladium.

Un procédé particulièrement préféré est celui qui a été décrit dans le document US-A 4.845.208, dont la Demanderesse est titulaire, qui consiste à utiliser comme catalyseur d'oxydation du palladium fixé sur charbon actif et dopé au bismuth.

On peut également envisager d'oxyder l'ose par voie électrolytique ou à l'aide d'hypobromite. On peut encore oxyder l'ose par voie microbiologique à l'aide de *Gluconobacter* ou *d'Aspergillus.*

Les acides aldariques peuvent être obtenus, de manière connue, par oxydation des oses correspondants en présence d'air et de platine.

Dans le cadre de la présente invention, il n'y a aucune contrainte particulière en termes de matière sèche du milieu réactionnel. En pratique, le procédé selon l'invention est caractérisé par le fait que tout dérivé acide d'ose et/ou tout sel de dérivé acide d'ose est apporté, sous forme liquide et/ou solide, en quantité telle que la concentration totale en dérivé(s) acide(s) d'ose et sel(s) de dérivé(s) acide(s) d'ose, dans le milieu réactionnel, est comprise entre 1 et 90%, de préférence entre 20 et 90%, exprimée en poids sec par rapport au poids total du milieu réactionnel. Il est notamment remarquable, comme cela a été vérifié par la Société Demanderesse, que contrairement à ce qui est habituellement recommandé dans les réactions classiques de RUFF, il est possible ici de travailler à des matières sèches élevées, par exemple à une valeur comprise entre 30 et 60%, comme il est exemplifié ci-après.

Seules les contraintes de matières sèches minimales sont imposées pour des raisons évidentes d'économie d'évaporation d'eau et de réduction de la taille des réacteurs, des matières sèches de 1% comme utilisées dans le document EVERETT précité n'étant pas économiquement viables en pratique industrielle.

Dans la description ci-après, tous les pourcentages sont exprimés par rapport au total de dérivé(s) acide(s) d'ose et de sel(s) de dérivé(s) acide(s) d'ose, pouvant être présents dans le milieu réactionnel. (exemple : 50 mol % signifie 50 moles de X pour 100 moles, au total, de dérivé(s) acide(s) d'ose et de sel(s) de tel(s) dérivé(s) acide(s), et 50 % signifie 50 grammes de X pour 100 grammes, au total, de dérivé(s) acide(s) d'ose et de sel(s) de tel(s) dérivé(s) acide(s).

Le tungstène et/ou le molybdène utilisé(s) comme catalyseur(s) de la réaction peuvent être apportés, par exemple, sous forme d'oxydes (WO₃ ou MoO₃), d'acides (H₂WO₄ ou H₂MoO₄), d'hétéropolyacides de type KEGGIN ou DAWSON (acide phosphotungstique H₃PW₁₂O₄₀ ou acide phosphomolybdique H₃PMo₁₂O₄₀ par exemple), ou de sels (Na₂Wo₄, CaWo₄ ou Na₂MoO₄, CaMoO₄). Cependant, on préfère utiliser un sel de métal alcalin, alcalino-terreux ou d'ammonium du tungstène ou du molybdène comme, par exemple, le tungstate ou le molybdate de sodium, ou le molybdate d'ammonium.

Selon une variante du procédé conforme à l'invention, la quantité de sel(s) de tungstène et/ou molybdène en présence de laquelle se fait la réaction d'oxydation est inférieure à 1 équivalent, exprimée par le nombre total de mole(s) de tungstène et molybdène divisé par le nombre total de mole(s) de dérivé(s) acide(s) d'ose à n+1 atomes de carbone et de sel(s) de dérivé(s) acide(s) d'ose.

A titre d'exemple, lorsque le catalyseur est constitué uniquement de sel(s) de tungstène et que le substrat est constitué uniquement de GDL, une quantité de sel(s) de tungstène de 1 équivalent correspond à 1 mole de tungstène par mole de GDL soit, compte tenu des masses moléculaires respectives du tungstène (environ 184) et de la GDL (environ 178), à une quantité d'environ 184/178 soit d'environ 1,03 ou 103%, exprimée en poids de tungstène par rapport au poids de GDL.

Lorsque le catalyseur est constitué uniquement de sel(s) de molybdène, la masse moléculaire du molybdène étant de 96 environ, une quantité de sel(s) de molybdène de 1 équivalent correspond, par le même type de calcul, à une quantité d'environ 0,54 soit 54%, exprimée en poids de molybdène par rapport au poids de GDL.

Une quantité de sel(s) de tungstène et/ou de molybdène comprise entre 0,001 et 50 %, préférentiellement entre 0,001 et 20 % et plus particulièrement entre 0,001 et 10 % exprimée en poids de tungstène et/ou de molybdène par rapport au poids sec de dérivé(s) acide(s) d'ose mis en oeuvre et/ou de sel(s) de tel(s) dérivé(s) acide(s) d'ose donne de bons résultats dans le procédé conforme à l'invention, tant en ce qui concerne le rendement, la sélectivité que la pureté de l'aldose ou du dérivé d'aldose. On peut ajouter également au moins un agent dopant tel que écrit ci-avant comme du sulfate ou du chlorure de cuivre et ce, à une concentration totale comprise entre 0,0001 et 1%, de préférence comprise entre 0,001 et 0,5%, et par exemple de 0,05%, cette concentration étant exprimée en poids sec d'agent(s) dopant(s) par rapport au poids sec total de dérivé(s) acide(s) d'ose et de sel(s) de dérivé(s) acide(s) d'ose, pouvant être présents dans le milieu réactionnel.

Au mélange de dérivé(s) acide(s) d'ose et/ou de sel(s) associé(s), de catalyseur et d'eau, on ajoute lentement, sous agitation, le peroxyde d'hydrogène, de préférence sous forme d'eau oxygénée d'une richesse de 25 % à 70 %, à raison de 1 à 500 mol %, plus particulièrement de 50 à 300 mol % par rapport au dérivé acide d'ose mis en oeuvre ou à l'un de ses sels.

Le procédé conforme à l'invention est mis en oeuvre à un pH compris entre 1 et 8, dépendant de la forme (acide, éventuellement lactonisée et/ou de sel(s)) de tout dérivé acide d'ose introduit dans le milieu réactionnel.

De préférence, le procédé de l'invention est mis en oeuvre à une température comprise entre 0 et 100°C, préférentiellement comprise entre 20 et 90°C, et notamment comprise entre 20 et 50°C environ.

L'addition de l'eau oxygénée est effectuée à une vitesse d'introduction telle que la température du milieu réactionnel ne s'élève pas de préférence au-delà de 60°C. Ainsi, la vitesse d'introduction de l'eau oxygénée se situe généralement entre 30 minutes et 20 heures, fonction de l'exothermie de la réaction.

Généralement, des températures inférieures à 20°C amènent des temps de réaction trop longs, et des températures supérieures à 90°C, outre qu'elles nécessiteraient l'emploi de réacteurs résistants à la pression, conduiraient à une dégradation des produits de la réaction.

L'invention sera mieux comprise au moyen des quatre exemples qui suivent et qui ont pour seul but de mieux illustrer l'invention sans vouloir la réduire aux modes de réalisation expressément décrits et au dérivé acide d'ose mis en oeuvre.

Dans les exemples qui suivent, tous les résultats sont exprimés en pourcentage molaire.

### EXEMPLE 1

De la glucono-δ-lactone (91,9 grammes, 0,516 mole), du tungstate de sodium dihydrate (0,5 grammes, 1,5 mmoles) et de l'eau (500 ml) sont introduits dans un réacteur double enveloppe. Le mélange est porté à une température de 50°C. L'eau oxygénée à 30% (110 ml, 1,1 moles) est introduite en 90 minutes sans réguler le pH de la réaction. A la fin de l'addition, la solution est agitée jusqu'à la disparition complète des peroxydes, soit 30 heures. Le mélange réactionnel est enfin refroidi.

Le taux de conversion est de 48% et le rendement molaire en D-arabinose s'établit à 30%.

La coproduction d'acide formique n'est seulement que de 18,5% (rendement molaire), alors que dans les conditions classiques de la réaction de RUFF, celle-ci est moins sélective et génère de 60 à 70% d'acide formique.

### EXEMPLE 2

Le mélange réactionnel est établi conformément à l'exemple 1, avec cependant l'ajoût de 50 mg de sulfate cuivrique pentahydrate.

On constate alors qu'après 1,5 heure de réaction, le taux de conversion atteint 93 %, et le rendement molaire en D-arabinose est de 80 %. En outre, aucune production significative d'acide formique n'est déplorée.

Il ressort de ce qui précède que l'utilisation d'un tel agent dopant est particulièrement favorable à l'efficacité de la réaction et ce, tant en termes de durée que de sélectivité de réaction.

La présente invention a donc également pour objet un procédé tel que décrit ci-avant, caractérisé par le fait qu'il est conduit en présence d'un, au moins, agent dopant choisi parmi les espèces métalliques, en particulier les sels métalliques, à base de métaux autres que le tungstène et le molybdène, de préférence choisi parmi les sels de cuivre.

### EXEMPLE 3

De la glucono-δ-lactone (184g, 1,03 moles), du molybdate de sodium dihydrate (0,8g, 0,0033 moles), du sulfate cuivrique pentahydrate (80mg) et de l'eau (275ml) sont introduits dans un réacteur double-enveloppe. Le mélange est porté à 50°C. L'eau oxygénée à 50% (77ml, 1,34 moles) est introduite en 120 minutes. La réaction est légèrement exothermique. A la fin de l'addition, l'agitation est poursuivie pendant 10 minutes et le mélange est ensuite refroidi. Le taux de conversion est de 84,6% et le rendement en D-arabinose de 73,6%. En outre, aucune production significative d'acide formique n'est également déplorée.

### EXEMPLE 4

De la glucono-δ-lactone (400 g, 2,24 moles), du gluconate de sodium (10g, 0,046 mole), du molybdate d'ammonium de formule (NH₄)₂ Mo₄O₁₃, 2H₂O (2,8 g dont 1,62g de molybdène soit 0,017 mole de molybdène), du sulfate cuivrique pentahydrate (0,4g) et de l'eau (1600 g) sont introduits dans un réacteur double enveloppe. Le mélange est porté à 30°C.

L'eau oxygénée à 30% (600 ml, 5,9 moles) est additionnée en 15 heures au total, cette addition étant entrecoupée, au bout de la sixième heure, par l'ajout de glucono-δ-lactone (400g) et de gluconate de sodium (10g) et l'homogénisation du milieu réactionnel pendant 20 minutes. En suite de quoi, l'introduction d'eau oxygénée est reprise et ce, pour les 9 heures restantes, puis le mélange est refroidi. Le rendement molaire en D-arabinose est de 91%.

Cet exemple montre que l'on peut avantageusement, dans le cadre de l'invention, utiliser un mélange contenant un dérivé acide d'ose, en l'occurrence de la glucono-δ-lactone (GDL) et un sel de dérivé acide d'ose, en l'occurrence du gluconate de sodium, comme substrat de la réaction d'oxydation par le peroxyde d'hydrogène. Dans le cas présent la quantité de sel de dérivé acide d'ose, à savoir 10g + 10g = 20g (sec) représente 2,5% de la quantité de dérivé acide d'ose, à savoir 400g + 400g = 800g (sec), mise en oeuvre au sein d'un tel mélange.

Cet exemple confirme que l'on peut obtenir une réaction d'oxydation avec des sélectivité et rendement insoupçonnés en mettant en oeuvre des quantités de catalyseur(s) (molybdène et/ou tungstène) extrêmement faibles, notamment en regard de celles préconisées dans le document EVERETT précité.

Dans le cas présent, le substrat est constitué au total de 2 x (2,24 + 0,046) soit 4,572 moles de dérivé acide d'ose (GDL) et de sel de dérivé acide d'ose (gluconate de sodium). L'oxydation est effectuée en présence de 0,017 mole de molybdène comme indiqué ci-dessus et donc de 0,017/4,572 soit 0,0037 équivalent de molybdène environ. En poids, cette concentration correspond à 1,62g de molybdène pour 820g de substrat (GDL + gluconate de sodium), soit 0,2% environ de molybdène par rapport au poids total de dérivé acide d'ose et de sel de dérivé acide d'ose.

Pour sa part, le sulfate de cuivre pentahydrate est ici introduit à une concentration de 0,4g pour 820g de substrat soit d'environ 0,05% et donc environ 4 fois plus faible encore que la concentration pondérale du molybdène (0,2%).

Cet exemple confirme l'intérêt d'associer les catalyseurs utilisés conformément à l'invention, à savoir le tungstène et le molybdène, à des agents dopants tels que les sels de cuivre.

## Revendications

1. Procédé de fabrication d'un aldose ou d'un dérivé d'aldose comportant sur la chaîne hydrocarbonée n atomes de carbone, caractérisé par le fait que l'on met en contact, en phase aqueuse, au moins un dérivé acide d'ose à n + 1 atomes de carbone comportant au moins un motif α-hydroxyacide, et/ou au moins un sel d'un tel dérivé acide d'ose, avec du peroxyde d'hydrogène en présence d'une quantité d'au moins un sel de tungstène ou de molybdène, ladite quantité étant inférieure à 4 équivalents, de préférence inférieure à 2 équivalents, exprimée en nombre total de mole(s) de tungstène et molybdène divisé par le nombre total de mole(s) de dérivé(s) acide(s) d'ose et de sel(s) de dérivé(s) acide(s) d'ose.

2. Procédé de fabrication selon la revendication 1, caractérisé par le fait que tout dérivé acide d'ose et/ou tout sel de dérivé acide d'ose est apporté, sous forme liquide et/ou solide, en quantité telle que la concentration totale en dérivé(s) acide(s) d'ose et sel(s) de dérivé(s) acide(s) d'ose, dans le milieu réactionnel, est comprise entre 1 et 90%, de préférence entre 20 et 90%, exprimée en poids sec par rapport au poids total de milieu réactionnel.

3. Procédé de fabrication selon l'une des revendications 1 et 2, caractérisé par le fait que la quantité de sel(s) de tungstène et de molybdène est inférieure à 1 équivalent, exprimée en nombre total de mole(s) de tungstène et molybdène divisé par le nombre total de mole(s) de dérivé(s) acide(s) d'ose et de sel(s) de dérivé(s) acide(s) d'ose.

4. Procédé de fabrication selon la revendication 3, caractérisé par le fait que la quantité de sel(s) de tungstène et de molybdène est comprise entre 0,001 et 50%, de préférence entre 0,001 et 20%, et plus particulièrement entre 0,001 et 10%, exprimée en poids de tungstène et de molybdène par rapport au poids sec de dérivé(s) acide(s) d'ose et de selfs) de dérivé(s) acide(s) d'ose, mis en oeuvre.

5. Procédé de fabrication selon l'une quelconque des revendications 1 à 4, caractérisé par le fait que l'on met en oeuvre, en tant que substrat mis en contact avec le peroxyde d'hydrogène, un mélange contenant au moins un dérivé acide d'ose, sous forme libre et/ou lactonisée et au moins un sel, de préférence alcalin ou alcalino-terreux, d'un dérivé acide d'ose.

6. Procédé de fabrication selon la revendication 5, caractérisé par le fait que le poids sec de sel(s) de dérivé(s) acide(s) d'ose au sein du mélange représente de 0,5 à 20%, de préférence de 1 à 10%, du poids sec de dérivé(s) acide(s) d'ose contenu dans ledit mélange.

7. Procédé de fabrication selon l'une quelconque des revendications 1 à 6, caractérisé par le fait que le dérivé acide d'ose est un acide aldonique, de préférence l'acide gluconique, sous forme libre et/ou lactonisée et que le sel de dérivé acide d'ose est un sel d'acide aldonique, de préférence d'acide gluconique et plus particulièrement un sel alcalin ou alcalino-terreux d'acide gluconique.

8. Procédé de fabrication selon l'une quelconque des revendications 1 à 7, caractérisé par le fait que le sel de tungstène ou de molybdène est choisi dans le groupe comprenant les oxydes, les acides, les hétéropolyacides, les sels de métaux alcalins, les sels de métaux alcalino-terreux et les sels d'ammonium, de préférence choisi parmi les sels alcalins, alcalino-terreux et d'ammonium, du tungstène et du molybdène.

9. Procédé de fabrication selon l'une quelconque des revendications 1 à 8, caractérisé par le fait qu'il est conduit en présence d'au moins un agent dopant choisi parmi les espèces métalliques, en particulier les sels métalliques, à base de métaux autres que le tungstène et le molybdène, de préférence choisi parmi les sels de cuivre.

10. Procédé de fabrication selon la revendication 9, caractérisé par le fait qu'il est conduit en présence de 0,0001 à 1%, de préférence de 0,001 à 0,5%, d'agent(s) dopant(s), cette concentration étant exprimée en poids sec total d'agent(s) dopant(s) par rapport au poids sec total de dérivé(s) acide(s) d'ose et de sel(s) de dérivé(s) acide(s) d'ose présents dans le milieu réactionnel.
